(19) Europäisches Patentamt
European Patent Office
Office européen des brevets

(11) Publication number: **0 135 252**
**A2**

(12) **EUROPEAN PATENT APPLICATION**

(21) Application number: 84304007.2

(22) Date of filing: 14.06.84

(51) Int. Cl.[4]: **C 07 C 121/45**
C 07 C 120/00, C 07 C 121/34
C 07 C 121/00, C 07 C 121/66

(30) Priority: 20.06.83 US 506175

(43) Date of publication of application:
27.03.85 Bulletin 85/13

(84) Designated Contracting States:
BE CH DE FR GB IT LI LU NL SE

(71) Applicant: ELI LILLY AND COMPANY
307, East McCarty Street
Indianapolis Indiana 46285(US)

(72) Inventor: Hackler, Ronald Ervin
419 Woodland West Drive, Rt. 7
Greenfield Indiana 46140(US)

(72) Inventor: Wickiser, David Ira
8140 Rumford Road
Indianapolis Indiana 46219(US)

(74) Representative: Tapping, Kenneth George et al,
Lilly Industries Limited Patent Department Erl Wood Manor
Windlesham Surrey, GU20 6PH(GB)

(54) **Process for preparing 3-substituted-3-aminonitrile.**

(57) A novel process for preparing aminonitriles is described. Some of the aminonitriles are novel. All the aminonitriles serve as intermediates for preparing isothiazolylurea compounds which are useful as herbicides and algicides.

EP 0 135 252 A2

Croydon Printing Company Ltd

PROCESS FOR PREPARING
3-SUBSTITUTED-3-AMINONITRILE

This invention concerns a novel synthesis for 3-substituted-3-aminonitriles, which are useful as intermediates to 3-substituted-5-isothiazolylureas. These urea compounds are useful as algicides, and aquatic and terrestrial herbicides. In addition, some of the 3-substituted-3-aminonitriles are novel compounds.

Alternative synthesis known in the prior art for preparing some of the 3-substituted-3-aminonitriles are described in the following references.

Brown, D.J. et al. in "The Dimroth Rearrangement. Part XVI. A New General Synthesis and Rearrangement of C-Alkylated 1,6-Dihydro-6-imino-1-methylpyrimidines", J. Chem. Soc. Perkin I, 372-378 (1974) disclose aminonitriles or enaminonitriles, such as 3-amino-3-isopropylpropenenitrile. The aminonitriles were prepared by lithium aluminum hydride reduction of malononitrile or an appropriate C-alkyl derivative.

In Prelog, V. and Szpilfogel, S., "Cycloalkenopyridine. Pyrindan and Bz-Tetrahydro-chinolin", Helv. Chim. Acta, 28, 1684-1692 (1945), the authors describe the conversion of a ketone to an amine using ammonium nitrate and ammonia.

Lang, S. et al., "β-Aminocinnamonitriles as Potential Antiinflammatory Agents", J. Med. Chem., 18, 441-3 (1975) shows the preparation of an aminonitrile by reacting acetonitrile with an aromatic nitrile.

Beilstein's Handbuch der Organischen Chemie, 3, 660 describes the condensation of acetonitrile with itself to form the base $CH_3C(NH_2)=CHCN$. Beilstein 10, I, 321 describes the reaction of a benzonitrile with acetonitrile and sodium metal to form a 3-amino-3-substituted-propenenitrile.

The present process provides several improvements over the prior art. The 3-aminonitrile is provided in high yield without by-products, using reagents that are easily handed and relatively inexpensive, to provide some novel products which cannot be obtained by the prior art methods.

The present novel process concerns preparing an aminonitrile of the formula

$$R-\underset{NH_2}{\underset{|}{C}}=CH-CN \qquad III$$

wherein R is $C_1$-$C_{10}$ alkyl, $C_3$-$C_6$ cycloalkyl, ($C_1$-$C_4$ alkyl)$C_3$-$C_6$ cycloalkyl, ($C_2$-$C_4$ alkenyl)$C_3$-$C_6$ cycloalkyl, ($C_1$-$C_4$ haloalkyl)$C_3$-$C_6$ cycloalkyl, ($C_3$-$C_6$ cycloalkyl)$C_1$-$C_4$ alkyl, 2-thienyl, or

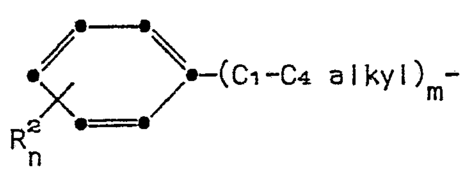

wherein $R^2$ is hydrogen, halo, $C_1$-$C_6$ alkyl, $C_1$-$C_3$ alkoxy, or trifluoromethyl; n is 1 or 2; and m is 0 or 1;

which comprises reacting a ketonitrile of the formula

$$R-\overset{O}{\overset{\|}{C}}-CH_2-CN \qquad II$$

wherein R is defined as above, with a source of ammonia.

The source of ammonia is at least one equivalent of ammonia either (1) in a sealed vessel at a temperature from about 100 to about 150°C or (2) in an inert organic solvent at a temperature from about 25 to about 80°C in the presence of an ammonium salt, other than ammonium sulfate.

Also within the scope of this invention are novel intermediates defined as follows:

A compound of the formula

$$R^3-\underset{NH_2}{\underset{|}{C}}=CH-C\equiv N \qquad IIIA$$

wherein

$R^3$ is $C_4$-$C_{10}$ alkyl, except $R^3$ cannot be 1-ethylpropyl; $C_3$-$C_6$ cycloalkyl; $(C_1$-$C_4$ alkyl)$C_3$-$C_6$ cycloalkyl; $(C_2$-$C_4$ alkenyl)$C_3$-$C_6$ cycloalkyl; $(C_1$-$C_4$ haloalkyl)$C_3$-$C_6$ cycloalkyl; or $(C_3$-$C_6$ cycloalkyl)$C_1$-$C_4$ alkyl.

More preferred compounds of formula IIIA are those wherein $R^3$ is $C_4$-$C_6$ alkyl, $C_3$-$C_6$ cycloalkyl, ($C_1$-$C_4$ alkyl)$C_3$-$C_6$ cycloalkyl, or ($C_3$-$C_6$ cycloalkyl)-$C_1$-$C_4$ alkyl.

The following are representative of the intermediate compounds of formula III:

3-amino-3-cyclopentylpropenenitrile
3-amino-3-cyclohexylpropenenitrile
3-amino-3-(1-methylcyclohexyl)propenenitrile
3-amino-4-cyclohexyl-2-butenenitrile
3-amino-2-pentenenitrile
3-amino-4-methyl-2-pentenenitrile
3-amino-4,4-dimethyl-2-pentenenitrile
3-amino-4,4-diethyl-2-pentenenitrile
3-amino-2-hexenenitrile
3-amino-4-methyl-2-hexenenitrile
3-amino-5-methyl-2-hexenenitrile and
3-amino-2-heptenenitrile.

Preferred compounds are:

3-amino-3-cyclohexylpropenenitrile
3-amino-3-(1-methylcyclohexyl)propenenitrile
3-amino-3-(3-chlorophenyl)propenenitrile
3-amino-3-(4-fluorophenyl)propenenitrile
3-amino-3-(3-methylphenyl)propenenitrile
3-amino-4-cyclohexyl-2-butenenitrile and
3-amino-4-methyl-2-pentenenitrile.

The following defines the various terms used in the above formulae and those formulae in the below process scheme.

The term "$C_1$-$C_{10}$ alkyl" refers to the straight and branched aliphatic radicals of one to ten carbon atoms including ethyl, propyl, isopropyl (1-methylethyl), butyl, methyl, isobutyl (2-methylpropyl), sec-butyl (1-methylpropyl), tert-butyl (1,1-dimethylethyl), pentyl, isopentyl (3-methylbutyl), sec-pentyl (1-methylbutyl), 1,1-dimethylpropyl, 1,2-dimethylpropyl, neopentyl (2,2-dimethylpropyl), hexyl, isohexyl (4-methylpentyl), sec-hexyl (1-methylpentyl), 2-methylpentyl, 3-methylpentyl, 1,1-dimethylbutyl, 2,2-dimethylbutyl, 3,3-dimethylbutyl, 1,2-dimethylbutyl, 1,3-dimethylbutyl, 1,2,2-trimethylpropyl, 1,1,2-trimethylpropyl, 1-methyl-1-ethylpropyl, heptyl, isoheptyl (5-methylhexyl), sec-heptyl (1-methylhexyl), 1-ethylpentyl, 2,2-dimethylpentyl, 3,3-dimethylpentyl, 4,4-dimethylpentyl, 1,2-dimethylpentyl, 1,4-dimethylpentyl, 1,2,3-trimethylbutyl, 1,1,2-trimethylbutyl, 1,1,3-trimethylbutyl, octyl, 1-methylheptyl, 2-methylheptyl, 3-methylheptyl, 4-methylheptyl, 1,1-dimethylhexyl, 2,2-dimethylhexyl, 3,3-dimethylhexyl, 4,4-dimethylhexyl, 5,5-dimethylhexyl, 1,2-dimethylhexyl, 1,3-dimethylhexyl, 1,4-dimethylhexyl, 1,2,3-trimethylpentyl, 1,1,2-trimethylpentyl, nonyl, 1-methyloctyl, 2,2-dimethylheptyl, 1,2,3-trimethylhexyl, 1,2,3,4-tetramethylpentyl, decyl, and the like. The terms "$C_2$-$C_{10}$ alkyl", "$C_1$-$C_6$ alkyl", "$C_2$-$C_6$ alkyl", "$C_1$-$C_5$ alkyl", "$C_3$-$C_4$ alkyl", "$C_1$-$C_4$ alkyl", and "$C_1$-$C_3$ alkyl" are also included in this definition.

The term "$C_1$-$C_3$ alkoxy" refers to the straight and branched aliphatic radicals of one to three carbon

atoms attached to the remainder of the molecule by an oxygen atom. Such alkoxys include methoxy, ethoxy, and propoxy.

The term "$C_3$-$C_6$ cycloalkyl" refers to saturated aliphatic rings of three to six carbon atoms, such as cyclopropyl, methylcyclopropyl, cyclobutyl, cyclopentyl, cyclohexyl, and the like.

The term "$C_1$-$C_4$ haloalkyl" refers to the straight and branched aliphatic radicals of one to four carbon atoms which have a halogen atom attached to the alkyl. The halogens include bromide, chloride, fluoride, and iodide.

The term "halo" refers to bromo, chloro, fluoro, and iodo.

## Process

The process is also described in the following schematic:

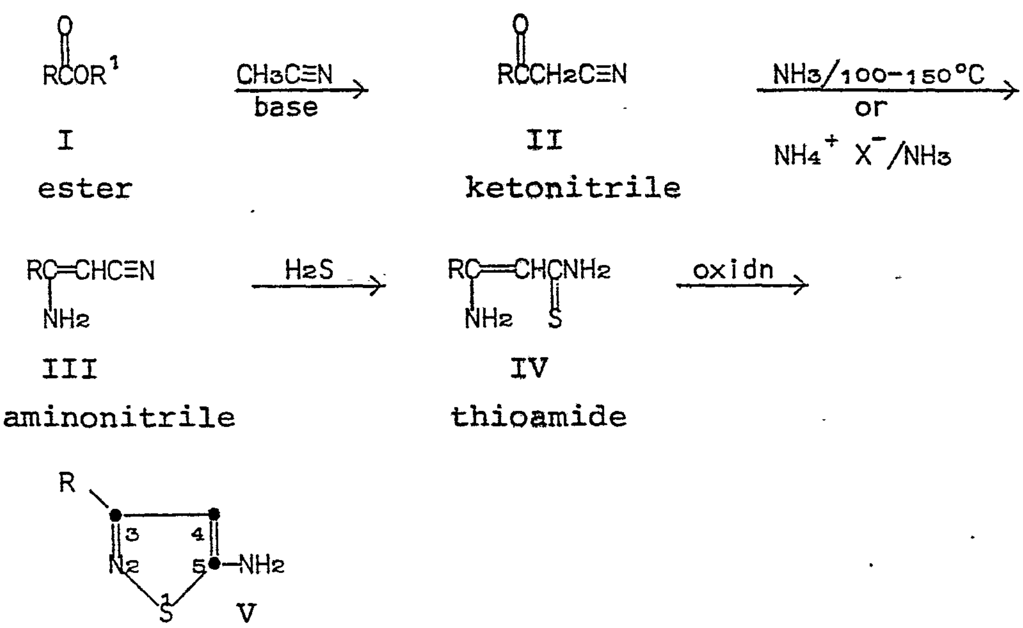


I ester

II ketonitrile

III aminonitrile

IV thioamide

V isothiazole

wherein

R is $C_1$-$C_{10}$ alkyl, $C_3$-$C_6$ cycloalkyl, ($C_1$-$C_4$ alkyl)$C_3$-$C_6$ cycloalkyl, ($C_2$-$C_4$ alkenyl)$C_3$-$C_6$ cycloalkyl, ($C_1$-$C_4$ haloalkyl)$C_3$-$C_6$ cycloalkyl, ($C_3$-$C_6$ cycloalkyl)$C_1$-$C_4$ alkyl, 2-thienyl, or

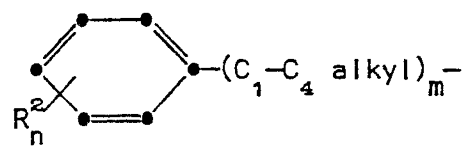 ;

m is 0 or 1;

$R^2$ is hydrogen, halo, $C_1$-$C_6$ alkyl, $C_1$-$C_3$ alkoxy, or trifluoromethyl;

n is 1 or 2; and

$R^1$ is $C_1$-$C_6$ alkyl, or

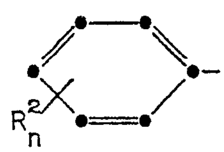 ; and

X is nitrate, acetate, formate, bromide, chloride, or fluoride.

In the above process, the claimed novel process is shown by the reaction of the ketonitrile II → the aminonitrile III. The intermediates of formula IIIA are also novel. The preparation of the starting compound of formula II is shown by the reaction of the ester I → ketonitrile II. Subsequent intermediates are prepared by the reactions of the aminonitrile III → thioamide IV → isothiazole V.

Each of these compound formulae are discussed below.

Compound I - Ester

Compound I, the substituted ester, can be obtained by methods well-known in the art. These include the preparation of the ester from the corresponding carboxylic acid, RCOOH, wherein R is defined above, and the appropriate alcohol, $R^1OH$, wherein $R^1$ is defined above, under acidic conditions. Typical acids used include concentrated sulfuric acid, dry hydrogen chloride, and boron trifluoride etherate, with boron trifluoride etherate preferred. Preferred alcohols are methanol and ethanol, wherein $R^1$ is methyl or ethyl, with the most preferred being methanol. The resulting preferred ester will be the methyl carboxylate of the acid. Preferred acid groups, R, are listed above. Usually, to shift the equilibrium toward the ester, a large excess of alcohol is used with elevated temperatures, but the temperature can be from about ambient temperature to about the reflux temperature of the alcohol. Alternatively, the resulting ester can be removed upon formation.

A more preferred preparation of the ester involves using the corresponding acid chloride, RCOCl, and the appropriate alcohol, $R^1OH$. Esterification of an aromatic acid chloride is often carried out in the presence of a base, such as pyridine, aqueous sodium hydroxide, or others, under conditions described as the Schotten-Baumann technique. However, sometimes the use of the above-listed bases is unnecessary. (Typically,

the acid chloride is prepared from its carboxylic acid using thionyl chloride, phosphorus trichloride, or phosphorus pentachloride.) Acid anhydrides can also undergo alcoholysis to form an ester, as do the acid chlorides.

The process called transesterification allows conversion of one ester to another, catalyzed by acidic or basic conditions. The acid can be concentrated sulfuric acid, dry hydrogen chloride, and the like, while the base can be an alkoxide ion, and the like. To shift the equilibrium to form the new ester, a large excess of the appropriate alcohol is used or the desired ester is removed upon formation.

Compound II - Ketonitrile

The ketonitrile, compound II, is formed from the substituted ester using a base condensation reaction with acetonitrile. The base present can be sodium metal, sodium ethoxide, sodium methoxide, sodium hydride, and the like. However, sodium hydride is the base of choice and is usually dispersed in mineral oil. The substituted ester is allowed to react with from about one to about two equivalents of acetonitrile in the presence of this base and a solvent at the reflux temperature of the reaction mixture. The reaction continues for a time sufficient to bring about substantial completion, usually from about 4 hours to about 24 hours. Suitable solvents include tetrahydrofuran (THF), ether, toluene, benzene, ethanol, methanol, and the like, with THF the preferred solvent.

A preferred method for preparing Compound II is slurrying sodium hydride in THF and then adding the appropriate ester and acetonitrile, while the slurry is maintained at a temperature from about 60 to about 65°C. After the addition is complete, the mixture is heated to reflux for about 16 to about 24 hours.

Compound III - Aminonitrile

Once the ketonitrile is formed, it can be converted to the aminonitrile, Compound III, by using a source of ammonia. One source of ammonia is using ammonia under pressure, with the pressure being generated by the reaction itself run in a sealed vessel. The reaction is heated at temperatures from about 100 to about 150°C for anywhere from about 4 to about 24 hours. Preferably, the reaction is conducted at about 150°C for about 16 hours. Although an organic solvent can be used in the reaction, such as ethanol, benzene, toluene, methanol, THF, and the like, with ethanol or other inert polar solvents being preferred, such a solvent is not essential to the reaction. It is, however, necessary to use at least one equivalent of ammonia, but an excess of ammonia can be used if desired.

The preferred source of ammonia utilizes an ammonium salt and ammonia under atmospheric pressure at temperatures from about ambient temperature to about the reflux temperature of the solvent used. The ketonitrile, Compound II, is mixed in an inert organic solvent that already contains the ammonium salt.

Typical solvents include ethanol, methanol, THF, and the like, with ethanol being preferred. Typical ammonium salts include ammonium chloride, ammonium bromide, ammonium acetate, ammonium formate, ammonium nitrate, and the like, with ammonium nitrate being preferred, but, ammonium sulfate cannot be used. The amount of ammonium salt can be varied and an excess can be used; however, it is preferable to use less than or equal to one equivalent of the salt in the reaction. The resulting mixture of solvent and ammonium salt is then warmed to the desired temperature, which is usually the reflux temperature of the solvent, and ammonia is bubbled in. The reaction runs from about 4 to about 24 hours, with the preferred time being from about 16 to 24 hours.

Alternatively, Compound II may be reacted with one to two equivalents of ammonia with an ammonium salt added, all under pressure, at a temperature of about 25 to about 80°C for about 4 to about 24 hours.

A more preferred method of preparing Compound III uses ammonium nitrate as the desired ammonium salt, uses ethanol as the solvent, and is run at the reflux temperature of ethanol, which is about 80°C, for about 16 hours.

Compound IV - Thioamide

Compound IV, a thioamide, can be made under pressure by following a procedure similar to that outlined in J. Goerdeler and H.W. Pohland, Chem. Ber., 94, 2950 (1961) and British Patent 1,153,186 (Netherlands Patent 6,608,094).

The aminonitrile, Compound III, is reacted with hydrogen sulfide under pressure; the pressure being generated by the reaction itself. This reaction is run in an inert organic solvent, such as methylene chloride, chloroform, and the like, with methylene chloride being preferred. A small amount of a base-catalyst (0.5-2%) is used to facilitate reaction and includes such bases as: triethylamine and potassium hydroxide, with the latter preferred. The temperature of the reaction can be from about 25 to about 100°C, with about 60°C being preferred, while the reaction can be allowed to continue from about 16 hours to about 72 hours, with about 48 hours being preferred.

A more preferred method of making the thioamide with hydrogen sulfide utilizes a temperature at about 60°C for about 48 hours, with methylene chloride as the solvent, and potassium hydroxide as the catalyst.

Alternatively, the thioamide can be prepared at atmospheric pressure by reacting the aminonitrile in an inert organic solvent such as pyridine, ethanol, toluene, benzene, and the like, pyridine being preferred, with hydrogen sulfide in the presence of a base-catalyst, such as triethylamine. Typically this type of reaction is run at a temperature from about 25 to about 100°C.

Another route, even more preferred, involves using a phase-transfer catalyst (PTC) at about one to two atmospheres of pressure (atm), following a procedure similar to that outlined in L. Cassar, S.

Panossian, and C. Giordano, Synthesis, 917 (1978). The aminonitrile is dissolved in an inert organic solvent, such as benzene, toluene, 1,2-dichlorobenzene, diphenyl ether, and the like. (The use of a solvent can be avoided if a liquid aminonitrile is utilized.) Then an aqueous solution of sulfide ion (usually sodium sulfide nonahydrate is used to supply the sulfide ion) and the PTC are added. The use of dilute aqueous sulfide solutions are preferred, allowing the reaction to proceed more rapidly. The mixture is then heated to about 35-80°, preferably 70°C under an atmosphere of hydrogen sulfide at a pressure of about 1-2 atm for about 4 to about 48 hours. Typical PTCs include tetrabutylammonium chloride, tetrabutylammonium bromide, tricaprylmethylammonium chloride, dibenzo-18-crown-6, and the like. The preferred PTC is tetrabutylammonium chloride (TBAC).

Compound V - Isothiazole

Ring closure of the thioamide to form the isothiazole is facilitated by using an oxidizing agent, such as iodine, bromine, hydrogen peroxide, sodium hypochlorite, chloramine, chloramine T, chlorine, persalts such as potassium or ammonium persulfate, and the like. Preferred oxidizing agents are iodine and hydrogen peroxide. First, the thioamide is dissolved in an inert organic solvent, such as ether, benzene, ethanol water, and the like, and then the oxidizing agent is added.

Isolation of Intermediates

Each intermediate formed can be isolated, if desired, by typical techniques, such as extraction or recrystallization. However, isolation is not necessary when an intermediate is going to be used in a following reaction. Compound I is typically isolated by being washed with water and then extracted with ether and dried over magnesium sulfate.

The ketonitrile, Compound II, is formed in the reaction mixture as the sodium salt. Then the solvent is removed *in vacuo* before the salt is dissolved in water. The residual mineral oil is washed out with hexane, and the free ketonitrile is obtained by acidifying with a mineral acid, such as hydrochloric acid and the like, and extracting into ether. Any necessary crystallization can be done with ethanol, methylene chloride, cyclohexane, hexane, toluene, and the like, or appropriate mixtures thereof.

Compound III, the aminonitrile, can be isolated by removing the excess ammonia and solvent *in vacuo*, and then extracting with hexane, methylene chloride, ethyl acetate, ether, and the like. The mixture containing the product can also be dried over magnesium sulfate and then recrystallized. Typical solvents used for crystallization are toluene, hexane, cyclohexane, and the like.

The thioamide, prepared by the first route, can be isolated by removing the excess hydrogen sulfide and solvent and then crystallizing it from solvents,

such as ethanol, toluene, benzene, ethyl acetate, chloroform, hexane, and the like. Once the preparation of the thioamide is completed using the PTC route, the water layer is removed, and the organic layer is isolated. The product contained in the organic layer is precipitated, and can be recrystallized from solvents, such as methylene chloride, ether, benzene, hexane, ethyl acetate, and the like. Any excess solvent can be removed and/or dried before the desired product is obtained.

After cyclization of the thioamide, the isothiazole can be isolated as follows. The organic layer containing the product can be dried and then the solvent removed. Crystallization can be done using toluene, ethanol, cyclohexane, acetone, hexane, methylene chloride, chloroform, ether, and mixtures thereof, and the like.

When impure thioamide is used in the cyclization step, the isothiazole product is best purified by extracting it from the organic solvent with a dilute mineral acid, washing the aqueous solution with an organic solvent, such as methylene chloride, and the like. The isothiazole is then freed with sodium or potassium hydroxide, and can be collected by filtration if it is a solid or it can be extracted into an inert organic solvent, such as methylene chloride and the like. Then the solution is dried and the solvent removed.

The following examples are illustrative of the claimed process and intermediates, but are not to be construed as limitations on it. All temperatures are in degrees Celsius and melting points are uncorrected.

STARTING MATERIALS

Examples of preparation of Compound I

Example A

Ethyl 1-methylcyclohexylcarboxylate

One hundred grams (0.704 mole) of 1-methylcyclohexylcarboxylic acid and 97.02 g (0.77 mole) of oxalyl chloride (ethandioyl chloride) were mixed in 750 ml of carbon tetrachloride and stirred at room temperature until all hydrogen chloride gas evolution stopped. The carbon tetrachloride and excess oxalyl chloride were removed, and the crude 1-methylcyclohexylcarboxyl chloride formed was added to 750 ml of ethanol. After stirring at room temperature for about 16 hours, the excess ethanol was removed and the product, ethyl 1-methylcyclohexylcarboxylate, was collected. (Yield = 45%)

It was then vacuum distilled at .15 mm and the fraction coming off at 40-43° was collected. NMR ($CDCl_3$): δ4.1 (q, 2H); δ2.3-1.7 (m, 2H); δ1.7-1.0 (m, 15H).

## Example B

### Methyl cyclopentylcarboxylate

A mixture of 100 g of cyclopentylcarboxylic acid and 10 ml of boron trifluoride etherate was mixed in 500 ml of methanol and heated at reflux temperature for about 16 hours. After cooling, the mixture was poured into water and extracted with ether. Then the ether was removed, and the mixture was dried and distilled at atmospheric pressure.

The fraction distilling at about 150-155° was collected, yielding 75.2 g (67%).

The following elemental analysis was obtained:

Calculated for $C_7H_{12}O_2$:

Theory: C, 65.60; H, 9.44

Found: C, 65.32; H, 9.17.

The following examples were prepared using the general procedure of Example B.

## Example C

### Methyl 2-ethylbutanoate

BP (boiling point) = 134-140°

Yield = 72 g (55%)

NMR ($CDCl_3$): δ3.6 (s, 3H); δ2.4-1.8 (m, 1H); δ1.8-1.1 (m, 4H); δ0.8 (t, 6H).

## Example D

## Methyl 2-methylbutanoate

Yield = 30%
BP = 118-122°
Calculated for $C_6H_{12}O_2$:

Theory: C, 62.04; H, 10.41
Found: C, 61.80; H, 10.12.

## Example E

## Methyl 1-methylcyclopropylcarboxylate

Yield = 88 g (51%)
BP = 122-126°

## Example F

## Ethyl 2,4-dichlorobenzoate

About 200 g of 2,4-dichlorobenzoyl chloride was mixed in 700 ml of ethanol and stirred for about two hours. After the excess ethanol was removed, the resulting oil (yield = 20%) was submitted for NMR. NMR ($CDCl_3$): δ7.9-7.7 (d, 1H); δ7.5-7.1 (m, 2H); δ4.4 (q, 2H); δ1.45 (t, 3H).

The following example was prepared using the general procedure of Example F.

Example G

Ethyl 4-chlorobenzoate

Yield = 40%.

NMR ($CDCl_3$): δ8.2-7.7 (d, 2H); δ7.5-7.2 (d, 2H); δ4.4 (q, 2H); δ1.4 (t, 3H).

The following examples were prepared using the general procedure of Example F, except methanol was used as the solvent.

Example H

Methyl 3,3-dimethylbutanoate

Yield = 65 g (47%)

Calculated for $C_7H_{14}O_2$:

Theory: C, 64.58; H, 10.84

Found: C, 64.86; H, 10.53.

NMR ($CDCl_3$): δ3.8 (s, 3H); δ2.4 (s, 2H); δ1.2 (s, 9H).

Examples of preparation of Compound II

Example I

3-Ketoheptanenitrile

A dispersion of 76.8 g (1.6 mole) of 50% sodium hydride in mineral oil was added to 800 ml of tetrahydrofuran (THF). This solution was stirred under reflux as a solution of 104 g (0.8 mole) ethyl valerate and 65.6 g (1.6 mole) of acetonitrile in 250 ml of THF was added dropwise. After about one hour the foaming

became excessive, so the heat was removed and the solution was stirred overnight.

Then the solution was refluxed briefly and then cooled, followed by the dropwise addition of 40 ml of isopropyl alcohol. The solvent was then removed under vacuum. Six hundred ml of water was added and then extracted with 500 ml hexane and 500 ml of a hexane-ether mixture. A black material was filtered off and the solution acidified with concentrated hydrochloric acid and then extracted with two-300 ml portions of ether.

The combined extracts were washed with 150 ml of water and dried over magnesium sulfate before the solvent was removed under vacuum. The yield was 90 g of an oil (88%). NMR ($CDCl_3$): δ3.60 (s, 2H); δ2.60 (t, 2H); δ1.8-0.8 (m, 7H).

The following examples were prepared using the general procedure of Example I.

## Example J

## 3-Ketohexanenitrile

Yield = 89 g (96%)

NMR ($CDCl_3$): δ3.63 (s, 2H); δ2.60 (t, 2H); δ1.65 (m, 2H); δ0.97 (t, 3H).

Example K

4-Methyl-3-ketopentanenitrile

Yield = 76.5 g (86%)

BP = 95-100°/8 mm; 72-76°/1mm

NMR ($CDCl_3$): δ3.65 (s, 2H); δ2.8 (m, 1H); δ1.08 (d, 6H).

Example L

3-Cyclohexyl-3-ketopropionitrile

Yield = 94 g (89%)

BP = 95-100°/1 mm

Calculated for $C_9H_{13}NO$:

Theory: C, 71.49; H, 8.67; N, 9.26

Found: C, 71.52; H, 8.55; N, 9.42.

NMR ($CDCl_3$): δ3.55 (s, 2H); δ2.7-1.2 (m, 11H).

Example M

3-Ketopentanenitrile

Yield = 74 g (95%)

NMR ($CDCl_3$): δ3.63 (s, 2H); δ2.65 (q, 2H); δ1.1 (t, 3H).

Example N

3-(4-Chlorophenyl)-3-ketopropionitrile

Yield = 67%

MP (melting point) = 132-134°

Calculated for $C_9H_6ClNO$:

Theory: C, 60.19; H, 3.37; N, 7.80; Cl, 19.74

Found: C, 60.45; H, 3.18; N, 7.90; Cl, 19.52.

NMR ($CDCl_3$): δ8.1-7.7 (d, 2H); δ7.6-7.2 (d, 2H); δ4.3 (s, 2H).

Example O

4-Cyclohexyl-3-ketobutanenitrile

Yield = 61 g (89%)

NMR ($CDCl_3$): δ3.5 (s, 2H); δ2.5-0.6 (m, 13H).

Example P

3-(1-Methylcyclohexyl)-3-ketopropionitrile

BP = 112-115°/.7 mm

NMR ($CDCl_3$): δ3.8 (s, 2H); δ2.2-1.0 (m, 14H).

Example Q

3-(3-Trifluoromethylphenyl)-3-ketopropionitrile

Yield = 66 g (77%)

MP = 59-61°

Calculated for $C_{10}H_6F_3NO$:

Theory: C, 56.35; H, 2.84; N, 6.57; F, 26.74

Found: C, 56.29; H, 2.77; N, 6.49; F, 26.52.

Example R

4,4-Diethyl-3-ketopentanenitrile

Yield = 55.5 g (69%)

BP = 94-96°/1.5 mm

Calculated for $C_9H_{15}NO$:

Theory: C, 70.55; H, 9.87; N, 9.14

Found: C, 70.28; H, 9.95; N, 8.88.

Example S

4,4-Dimethyl-3-ketopentanenitrile

Yield = 82.9 g (83%)

MP = 60-62°

Example T

3-(1-Methylcyclopropyl)-3-ketopropionitrile

NMR ($CDCl_3$): δ3.7 (s, 2H); δ1.4 (m, 4H); δ0.9 (m, 3H).

Example U

4-Methyl-3-ketohexanenitrile

Yield = 57.77 g (77%)

BP = 49-52°/.1 mm

Example V

5,5-Dimethyl-3-ketohexanenitrile

Yield = 55.85 g (100%)

NMR ($CDCl_3$): δ3.8 (s, 2H); δ2.5 (s, 2H); δ0.9 (s, 9H).

Example W

4-Ethyl-3-ketohexanenitrile

Yield = 67 g (88%)

NMR ($CDCl_3$): δ3.65 (s, 2H); δ2.8 (m, 1H); δ1.7 (m, 4H); δ1.0 (t, 6H).

## Example X

### 3-Cyclopentyl-3-ketopropionitrile

Yield = 75.45 g (92%)

NMR ($CDCl_3$): δ3.8 (s, 2H); δ3.3-2.7 (m, 1H); δ2.1-1.5 (m, 8H).

## Example Y

### 5-Methyl-3-ketohexanenitrile

Yield = 99.93 g (89%)

NMR ($CDCl_3$): δ3.7 (s, 2H); δ2.7-1.7 (m, 3H); δ1.0 (d, 6H).

CLAIMED INTERMEDIATES

## Example 1

### 3-Amino-3-cyclohexylpropenenitrile

Ninety-one grams (0.6 mole) of 3-cyclohexyl-3-ketopropionitrile was added to 300 ml of liquid ammonia and 350 ml of ethanol and the mixture was heated in a sealed vessel at about 150° for about 16 hours. The solvent was removed under vacuum to leave an oil. Hexane was added to the oil and then the solution was cooled, leaving beige crystals with a MP of 44-47°. The product was recrystallized from hexane, giving a MP of 60-63°. The overall yield was 65 g (72%).

The following elemental analysis was obtained.

Calculated for $C_9H_{14}N_2$:

Theory: C, 71.96; H, 9.39; N, 18.65

Found: C, 72.17; H, 9.54; N, 18.90.

NMR ($CDCl_3$): δ4.7 (broad, 2H); δ3.7 (s, 1H); δ2.0-1.0 (m, 1H).

The following examples were prepared using the general procedure of Example 1.

Example 2

3-Amino-4,4-dimethyl-2-pentenenitrile

Yield = 93%

MP = 48-49°

NMR ($CDCl_3$): δ4.9 (broad, 2H); δ3.95 (s, 1H); δ1.2 (s, 9H).

Example 3

3-Amino-4-methyl-2-pentenenitrile

Yield = 52 g (70%)

NMR ($CDCl_3$): δ5.0 (broad, 2H); δ4.0 (s, 1H); δ3.75 (s, 1H); δ3.2-2.0 (m, 1H); δ1.15 (d, 6H).

Example 4

3-Amino-3-(4-chlorophenyl)propenenitrile

Yield = 34.75 g (72%)

MP = 130-132°

Calculated for $C_9H_7ClN_2$:

Theory: C, 60.52; H, 3.95; N, 15.68; Cl, 19.85

Found: C, 60.46; H, 4.03; N, 15.47; Cl, 20.13.

Example 5

3-Amino-4-cyclohexyl-2-butenenitrile

Yield = 24.5 g (55%)

NMR ($CDCl_3$): δ4.7 (m, 2H); δ3.7 (s, 1H); δ2.3-0.4 (m, 13H).

Example 6

3-Amino-3-(1-methylcyclohexyl)propenenitrile

Yield = 36.02 g (73%)

MP = 82-84°

Calculated for $C_{10}H_{16}N_2$:

Theory: C, 73.13; H, 9.82; N, 17.06

Found: C, 72.89; H, 9.67; N, 16.79.

Example 7

3-Amino-3-(3-trifluoromethylphenyl)propenenitrile

Yield = 35.15 g (52%)

MP = 60°

Calculated for $C_{10}H_7F_3N_2$:

Theory: C, 56.61; H, 3.33; N, 13.20; F, 26.86

Found: C, 56.50; H, 3.43; N, 13.03; F, 27.17.

Example 8

3-Amino-4,4-diethyl-2-pentenenitrile

Yield = 16.85 g (34%)

NMR ($CDCl_3$): δ5.3-4.5 (m, 2H); δ3.8 (s, 1H); δ1.5 (m, 4H); δ0.9 (m, 9H).

## Example 9

## 3-Amino-2-pentenenitrile

Yield = 25 g (50%)

BP = 80-85°/1 mm

## Example 10

## 3-Amino-4,4-dimethyl-2-pentenenitrile

Twelve and one-half grams (0.1 mole) of 4,4-dimethyl-3-ketopentanenitrile, 8.5 g (0.10 mole) of ammonium nitrate, and 100 ml of ethanol were stirred at room temperature as ammonia was bubbled into the solution. After 2.5 hours, TLC (methylene chloride) showed that product was present. The solution was then heated to reflux and the ammonia addition and refluxing were continued overnight.

One hundred-fifty ml of water was added and then the ethanol was removed under vacuum. The oil formed was extracted with 150 ml of ether, then was washed with 25 ml of water, and dried over magnesium sulfate. The ether was removed under vacuum, then hexane was added. Cooling the solution resulted in a pale yellow solid. The yield was 11.5 g (93%) with a MP of 48-49°.

The following elemental analysis was obtained:

Calculated for $C_7H_{12}N_2$:

Theory: C, 67.70; H, 9.74; N, 22.56

Found: C, 67.49; H, 9.57; N, 22.83.

The following examples were prepared using the general procedure of Example 10.

Example 11

3-Amino-4-methyl-2-pentenenitrile

Yield = 66.2 g (97%)

NMR ($CDCl_3$): δ5.0 (broad, 2H); δ4.0 (s, 1H); δ3.75 (s, 1H); δ3.2-2.0 (m, 1H); δ1.15 (d, 6H).

Example 12

3-Amino-2-hexenenitrile

Yield = 75 g oil (85%)

NMR ($CDCl_3$): δ5.2 (broad, 2H); δ4.1 (s, 1H); δ3.7 (s, 1H); δ2.8-0.9 (m, 7H).

Example 13

3-Amino-2-heptenenitrile

Yield = 81 g (90%)

NMR ($CDCl_3$): δ5.2 (broad, 2H); δ4.0 (s, 1H); δ3.7 (s, 1H); δ2.5-0.8 (m, 9H).

Example 14

3-Amino-4-ethyl-4-methyl-2-hexenenitrile

Yield = 59 g (97%)

MP = 45-48°

Calculated for $C_9H_{16}N_2$:

Theory: C, 71.01; H, 10.59; N, 18.40

Found: C, 71.09; H, 10.74; N, 18.18.

Example 15

3-Amino-4-methyl-2-hexenenitrile

Yield = 42.2 g (56%)

BP = 97-102°/.25-.5 mm

Calculated for $C_7H_{12}N_2$:

Theory: C, 67.71; H, 9.74; N, 22.56

Found: C, 67.47; H, 9.69; N, 22.41.

Example 16

3-Amino-5,5-dimethyl-2-hexenenitrile

Yield = 40.6 g (73%)

MP = 109-110°

Calculated for $C_8H_{14}N_2$:

Theory: C, 69.52; H, 10.21; N, 20.27

Found: C, 69.79; H, 9.97; N, 20.56.

Example 17

3-Amino-3-(1-methylcyclopropyl)-2-propenenitrile

Yield = 42.7 g (88%)

NMR ($CDCl_3$/DMSO): δ6.2 (s, 1H); δ5.8 (broad, 2H); δ1.4 (s, 3H); δ0.8 (m, 4H).

Example 18

3-Amino-2-pentenenitrile

Yield = 51 g (70%)

BP = 80-85°/1mm

Example 19

3-Amino-4-ethyl-2-hexenenitrile

Yield = 36.61 g (64%)

BP = 95-98°/.01 mm

Example 20

3-Amino-3-cyclopentylpropenenitrile

Yield = 85 g (100%)

Example 21

3-Amino-5-methyl-2-hexenenitrile

Yield = 62.75 g (63%)

NMR ($CDCl_3$): δ5.2 (broad, 2H); δ4.1 (s, 1H); δ3.6 (s, 1H); δ2.8-1.7 (m, 3H); δ1.0 (m, 6H).

SUBSEQUENT INTERMEDIATES

Examples of preparation of Compound IV

Example I

3-Amino-3-(4-chlorophenyl)propenethioamide

Seventy-three grams of 3-amino-3-(4-chlorophenyl)acrylonitrile and 500 ml of methylene chloride were added to 62 ml of liquid hydrogen sulfide and 1.0 g of potassium hydroxide. The reaction mixture was kept in a sealed vessel at about 80° for about 24 hours.

A yellow solid was collected and then washed with methylene chloride. This solid had a melting point greater than 245° and softened at about 185°.

The methylene chloride was removed from the filtrate under vacuum and then toluene was added to the residue. The resulting yellow solid had a MP of 176-181°. It was recrystallized from ethanol and then had a MP of 180-183°.

The first yellow solid was boiled in 700 ml ethanol and then filtered. Then about 250 ml of water was added to the filtrate, the solution was cooled, and a yellow solid was collected with a MP of 178-180°.

Both crops were combined to give 33 g (38% yield). The following elemental analysis was obtained:

Calculated for $C_9H_9ClN_2S$:

Theory: C, 58.82; H, 4.27; N, 13.17

Found: C, 58.89; H, 4.25; N, 12.99.

NMR (DMSO-$d_6$): δ8.2 (broad, 2H); δ7.3 (broad, 2H); δ6.8 (m, 5H); δ3.8 (s, 1H).

The following examples were prepared using the general procedure of Example I.

## Example II

### 3-Amino-3-cyclohexylpropenethioamide

Yield = 3.3 g (4%)

M.P. = 141-143°

Calculated for $C_9H_{16}N_2S$:

Theory: C, 58.65; H, 8.75; N, 15.20; S, 17.40

Found: C, 58.75; H, 9.00; N, 15.17; S, 17.61.

## Example III

3-Amino-4-cyclohexyl-2-butenethioamide

Yield = 12.25 g (69%)
MP = 89-91°

Calculated for $C_{10}H_{18}N_2S$:
Theory: C, 60.56; H, 9.15; N, 14.12; S, 16.17
Found: C, 60.48; H, 8.94; N, 13.85; S, 16.06.

## Example IV

3-Amino-3-(1-methylcyclohexyl)propenethioamide

Yield = 4 g (10%)

NMR ($CDCl_3$): δ8.0 (broad, 2H); δ6.3 (broad, 2H); δ2.3-0.7 (m, 13H)

## Example V

3-Amino-2-pentenethioamide

Yield = 19 g (59%)

NMR ($CDCl_3$): δ8.1 (broad, 2H); δ6.6 (broad, 2H); δ5.2 (5, 1H); δ2.2 (q, 2H); δ1.3 (t, 3H).

## Example VI

3-Amino-4,4-diethyl-2-pentenethioamide

Yield = 12.8 g (68%)

NMR ($CDCl_3$): δ8.0 (broad, 2H); δ6.7 (broad, 2H); δ5.2 (s, 1H); δ2.5-0.8 (m, 11H).

## Example VII

## 3-Amino-4,4-dimethyl-2-pentenethioamide

Yield = 58 g (59%)

NMR ($CDCl_3$): δ8.6-8 (m, 2H); δ6.6-5.8 (m, 2H); δ5.3 (s, 1H); δ1.2 (s, 9H).

## Example VIII

## 3-Amino-2-heptenethioamide

A solution of 81 g of 3-amino-2-heptenenitrile, 5 g of sodium sulfide nonahydrate ($Na_2S \cdot 9H_2O$), 3.8 g of tricaprylmethylammonium chloride, 100 ml of benzene, and 100 ml of water were stirred under an atmosphere of hydrogen sulfide at about 65-70° for about 16 hours.

The solution was cooled and extracted with 200 ml of ether. The extract was washed with 100 ml of water before drying over magnesium sulfate. The remaining solvent was removed under vacuum to leave 95 g of a dark oil. NMR indicated that approximately 50% of the desired product was formed. NMR ($CDCl_3$): δ8.1 (broad, 2H); δ6.5 (broad, 2H); δ5.2 (s, 1H); δ2.4-1.7 (m, 9H).

## Example IX

## 3-Amino-2-hexenethioamide

Seventy-five grams of 3-amino-2-hexenenitrile, 6 g of sodium sulfide nonahydrate, 3.4 g of tetrabutylammonium chloride, 100 ml of benzene, and 100 ml of water were stirred at about 65-70° under an

atmosphere of hydrogen sulfide for about 16 hours. The product was extracted with 400 ml of ether and the ether extract was washed with 100 ml of water. Then the extract was dried over magnesium sulfate and the ether was removed under vacuum, leaving a black oil. The yield was 84 g. NMR indicated the desired product was present to the extent of 25%. NMR ($CDCl_3$): δ8.0 (broad, 2H); δ6.6 (broad, 2H); δ5.1 (s, 1H); δ2.5-0.8 (broad, 7H).

The following examples were prepared using the general procedure of Example IX.

## Example X

### 3-Amino-3-cyclohexylpropenethioamide

Yield = 7.7 g (60%)

NMR ($CDCl_3$): δ8.0 (broad, 2H); δ6.2 (broad, 2H); δ2.3-0.6 (m, 11H).

## Example XI

### 3-Amino-4,4-dimethyl-2-pentenethioamide

Yield = 83.3 g

Second crop yield = 38.25 g (85%)

NMR ($CDCl_3$): δ8.6-8 (m, 2H); δ6.6-5.8 (m, 2H); δ5.3 (s, 1H); δ1.2 (s, 9H).

Example XII

3-Amino-3-(1-methylcyclopropyl)propenethioamide

NMR (DMSO) δ8.8-8.2 (broad, 2H); δ8.0-7.2 (broad, 2H); δ5.2 (s, 1H); δ1.3 (s, 3H); δ0.7 (d, 4H).

Example XIII

3-Amino-4-methyl-2-pentenethioamide

Yield = 77 g (89%)

NMR ($CDCl_3$): δ8.6-7.8 (broad, 2H); δ6.9-6.4 (broad, 2H); δ5.3 (s, 1H); δ2.3 (m, 1H); δ1.2 (d, 6H).

Example XIV

3-Amino-2-pentenethioamide

Yield = 46.85 g (68%)

NMR ($CDCl_3$): δ8.5 (broad, 2H); δ6.2 (broad, 2H); δ5.1 (s, 1H); δ2.1 (q, 2H); δ1.0 (m, 3H).

Example XV

3-Amino-3-cyclopentylpropenethioamide

Yield = 59.8 g (58%)

Example XVI

3-Amino-5-methyl-2-hexenethioamide

Yield = 62.5 g (80%)

NMR ($CDCl_3$): δ8.1 (broad, 2H); δ6.6 (broad, 2H); δ5.2 (s, 1H); δ2.7-1.8 (m, 3H); δ1.0 (m, 6H).

The following example was prepared using the general procedure of Example IX, except toluene was used instead of benzene.

Example XVII

3-Amino-4,4-dimethyl-2-pentenethioamide

Yield = 100 g (14%)

NMR ($CDCl_3$): δ8.6-8 (m, 2H); δ6.6-5.8 (m, 2H); δ5.3 (s, 1H); δ1.2 (s, 9H).

Examples of preparation of Compound V

Example XVIII

5-Amino-3-(4-chlorophenyl)isothiazole

A solution of 21.3 g (0.1 mole) of 3-amino-3-(4-chlorophenyl)propenethioamide, 29.2 g (0.21 mole) of potassium carbonate, and 1,000 ml of ether was stirred under reflux. Then 25.4 g (0.1 mole) of iodine in 250 ml of ether was added dropwise to the solution. Then it was stirred under reflux for 4 more hours and cooled. Five hundred ml of water was added, the resulting layers were separated, and the ether layer was dried over magnesium sulfate.

The ether was removed under vacuum leaving a yellow solid, which was then recrystallized from toluene. The MP was 128-129° and the yield was 17 g (81%).

The following elemental analysis was obtained:

Calculated for $C_9H_7ClN_2S$:

Theory: C, 51.33; H, 3.35; N, 13.30

Found: C, 51.54; H, 3.10; N, 13.26.

NMR (DMSO-$d_6$): δ7.95 (d, 2H); δ7.55 (d, 2H); δ6.8 (broad, 2H); δ6.75 (s, 1H).

The following examples were prepared using the general procedure of Example XVIII.

## Example XIX

### 5-Amino-3-cyclohexylisothiazole

MP = 132-136°

Calculated for $C_9H_{14}N_2S$:

Theory: C, 59.30; H, 7.74; N, 15.34

Found: C, 59.28; H, 7.91; N, 15.26.

NMR ($CDCl_3$): δ6.1 (s, 1H); δ4.5 (broad, 2H); δ2.6 (broad, 1H); δ2.2-1.2 (broad, 10H).

## Example XX

### 5-Amino-3-(1-methylethyl)isothiazole

Yield = 14.4 g (27%)

Second crop = 23.5 g (54%)

MP = 49-51°

Calculated for $C_6H_{10}N_2S$:

Theory: C, 50.64; H, 7.09; N, 19.70

Found: C, 50.44; H, 6.88; N, 19.50.

NMR ($CDCl_3$): δ6.1 (s, 1H); δ5.0 (broad, 2H); δ2.9 (m, 1H); δ1.25 (d, 6H).

Example XXI

5-Amino-3-propylisothiazole

Yield = 10.3 g (49%)

NMR ($CDCl_3$): δ6.0 (s, 1H); δ5.3 (broad, 2H); δ2.6 (t, 2H); δ1.6 (m, 2H); δ0.95 (t, 3H).

Example XXII

5-Amino-3-ethylisothiazole

Yield = 6.7 g (60%)

NMR ($CDCl_3$): δ6.1 (s, 1H); δ5.4 (broad, 2H); δ2.6 (q, 2H); δ1.2 (t, 3H).

Example XXIII

5-Amino-3-butylisothiazole

Yield = 16.6 g (36%)

NMR ($CDCl_3$): δ6.1 (s, 1H); δ5.0 (broad, 2H); δ2.6 (t, 2H); δ1.6 (m, 2H); δ1.4 (m, 2H); δ0.9 (t, 3H).

Example XXIV

5-Amino-3-(1-methylcyclohexyl)isothiazole

Yield = 70%

MP = 103-105°

Calculated for $C_{10}H_{16}N_2S$:

Theory: C, 61.18; H, 8.22; N, 14.27; S, 16.33

Found: C, 60.99; H, 7.93; N, 13.97; S, 16.45.

Example XXV

5-Amino-3-(1-methylcyclopropyl)isothiazole

Yield = 13.7 g (88%)

Example XXVI

5-Amino-3-(1-methylpropyl)isothiazole

Yield = 22.5 g (49%)

NMR ($CDCl_3$): δ6.0 (s, 1H); δ5.4-4.8 (broad, 2H); δ2.7 (m, 1H); δ1.6 (m, 2H); δ1.1 (d, 3H); δ0.9 (t, 3H).

Example XXVII

5-Amino-3-(1,1-dimethylethyl)isothiazole

Yield = 61 g (96%)

MP = 91-93°

Calculated for $C_7H_{12}N_2S$:

Theory: C, 53.81; H, 7.74; N, 17.93; S, 20.52

Found: C, 53.60; H, 7.45; N, 17.62; S, 20.50.

Example XXVIII

5-Amino-3-(1-ethyl-1-methylpropyl)isothiazole monohydrochloride

Yield = 34 g (67%)

MP = 140-142°

Calculated for $C_9H_{16}N_2S \cdot HCl$:

Theory: C, 48.97; H, 7.76; N, 12.69; S, 14.52; Cl, 16.06

Found: C, 48.94; H, 7.51; N, 12.83; S, 14.25; Cl, 16.32.

Example XXIX

5-Amino-3-(1-ethylpropyl)isothiazole

Yield = 6.1 g (35%)

NMR ($CDCl_3$): δ6.2 (s, 1H); δ5.4-4.7 (broad, 2H); δ2.6 (m, 1H); δ1.7 (m, 4H); δ0.9 (t, 6H).

Example XXX

5-Amino-3-(2-methylpropyl)isothiazole

Yield = 21 g (53%)

Calculated for $C_7H_{12}N_2S$:

Theory: C, 53.81; H, 7.74; N, 17.93; S, 20.52

Found: C, 53.82; H, 7.60; N, 17.69; S, 20.26.

NMR ($CDCl_3$): δ6.1 (s, 1H); δ5.0 (broad, 2H); δ2.5 (d, 2H); δ2.0 (broad, 1H); δ0.9 (t, 6H).

The compounds of formula V, 3-substituted-5-aminoisothiazoles, are useful as intermediates to 3-substituted-5-isothiazolylureas, which are useful as algicides, and aquatic and terrestrial herbicides. To prepare the ureas, the isothiazole compounds are usually converted to the carbamate and then reacted

with a substituted amine. The ureas can then be formulated with one or more aquatically- or agriculturally-acceptable carriers and applied to the infested area. Typical application rates are from about 0.01 to about 10 ppm of the urea compound for aquatic uses and from about 0.01 to about 10 pounds per acre (0.011 to about 11.2 kilograms per hectare) for terrestrial applications.

CLAIMS

1. A novel process for preparing an aminonitrile of the formula

$$R-\underset{NH_2}{\underset{|}{C}}=CH-CN \qquad III$$

wherein R is $C_1$-$C_{10}$ alkyl, $C_3$-$C_6$ cycloalkyl, ($C_1$-$C_4$ alkyl)$C_3$-$C_6$ cycloalkyl, ($C_2$-$C_4$ alkenyl)$C_3$-$C_6$ cycloalkyl, ($C_1$-$C_4$ haloalkyl)$C_3$-$C_6$ cycloalkyl, ($C_3$-$C_6$ cycloalkyl)$C_1$-$C_4$ alkyl, 2-thienyl, or

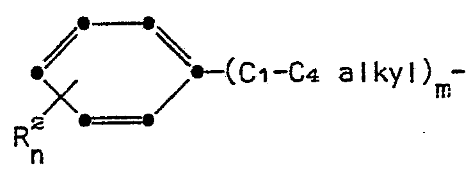

wherein $R^2$ is hydrogen, halo, $C_1$-$C_6$ alkyl, $C_1$-$C_3$ alkoxy, or trifluoromethyl; n is 1 or 2; and m is 0 or 1;

which comprises reacting a ketonitrile of the formula

$$R-\overset{O}{\overset{\|}{C}}-CH_2-CN \qquad II$$

wherein R is defined as above, with a source of ammonia.

2. A process of claim 1 wherein the compounds of formula III are represented by the subclass of compounds of the formula

$$R^3-\underset{\displaystyle NH_2}{C}=CH-C\equiv N \qquad \text{IIIA}$$

wherein

$R^3$ is $C_4$-$C_{10}$ alkyl, except $R^3$ cannot be 1-ethylpropyl; $C_3$-$C_6$ cycloalkyl; ($C_1$-$C_4$ alkyl)$C_3$-$C_6$ cycloalkyl; ($C_2$-$C_4$ alkenyl)$C_3$-$C_6$ cycloalkyl; ($C_1$-$C_4$ haloalkyl)$C_3$-$C_6$ cycloalkyl; or ($C_3$-$C_6$ cycloalkyl)$C_1$-$C_4$ alkyl.

3. A process of claim 1 or 2 wherein the source of ammonia is at least one equivalent of ammonia either (1) in a sealed vessel at a temperature from about 100 to about to about 150°C or (2) in an inert organic solvent at a temperature from about 25 to about 80°C in the presence of an ammonium salt, other than ammonium sulfate.

4. The process of claim 3 wherein the ketonitrile is treated with at least one equivalent of ammonia in a sealed vessel at a temperature from about 100 to about 150°C.

5. The process of claim 4 wherein an inert polar solvent is used.

6. The process of claim 5 wherein the solvent is ethanol.

7. The process of claim 3 wherein the ketonitrile is treated with at least one equivalent of ammonia in an organic solvent in the presence of an ammonium salt other than ammonium sulfate.

8. The process of claim 7 wherein the ammonium salt is ammonium nitrate.

9. The process of claim 8 wherein the solvent is ethanol, the reaction is run at about 80°C for about 16 hours, and one equivalent of ammonium nitrate is used.

10. The process of claim 8 wherein the reaction is run for about 4 to about 24 hours under pressure, using one to two equivalents of ammonia.

11. An aminonitrile of the formula

$$R^3\text{-}\underset{\displaystyle NH_2}{\underset{|}{C}}\text{=CH-C}\equiv\text{N} \qquad \text{IIIA}$$

wherein $R^3$ is

$C_4$-$C_{10}$ alkyl, except $R^3$ cannot be 1-ethylpropyl;

$C_3$-$C_6$ cycloalkyl;

($C_1$-$C_4$ alkyl)$C_3$-$C_6$ cycloalkyl;

($C_2$-$C_4$ alkenyl)$C_3$-$C_6$ cycloalkyl;

($C_1$-$C_4$ haloalkyl)$C_3$-$C_6$ cycloalkyl; or

($C_3$-$C_6$ cycloalkyl)$C_1$-$C_4$ alkyl.

12. Any one of the following compounds:

3-Amino-3-cyclohexylpropenenitrile

3-Amino-4,4-dimethyl-2-pentenenitrile

3-Amino-4-cyclohexyl-2-butenenitrile

3-Amino-3-(1-methylcyclohexyl)propenenitrile

3-Amino-4,4-diethyl-2-pentenenitrile

3-Amino-2-heptenenitrile

3-Amino-4-methyl-2-hexenenitrile

3-Amino-5,5-dimethyl-2-hexenenitrile

3-Amino-3-cyclopentylpropenenitrile

3-Amino-5-methyl-2-hexenenitrile

3-Amino-3-(1-methylcyclopropyl)propenenitrile.